# EUROPÄISCHE PATENTSCHRIFT

(11) **EP 1 903 019 B1**
(45) Veröffentlichungstag und Bekanntmachung des Hinweises auf die Patenterteilung: **02.09.2015**
(21) Anmeldenummer: 07112214.7
(22) Anmeldetag: 11.07.2007
(51) Int. Cl.: C07C 1/24, C07C 1/20

(54) **Verfahren zur Herstellung von Isoolefinen**
Method for the production of iso-olefines
Procédé de préparation d'isoléfines

(30) Priorität: 29.08.2006 DE 102006040433
(43) Veröffentlichungstag der Anmeldung: 26.03.2008
(73) Patentinhaber: Evonik Degussa GmbH, 45128 Essen (DE)
(72) Erfinder: Santiago Fernandez, Silvia, 33009, Oviedo (ES); Winterberg, Markus, Dr., 45711, Datteln (DE); Nierlich, Franz, Dr., 45768, Marl (DE); Houbrechts, Stephan, Dr., B-2570, Duffel (BE); Zanthoff, Horst-Werner, Dr., 45481, Mülheim a.d. Ruhr (DE); Büschken, Wilfried, Dr., 45721, Haltern am See (DE); Luh, Walter, 45770, Marl (DE); Skillas, Georg, Dr., 63456, Hanau (DE)

(56) Entgegenhaltungen:
- US-A- 4 447 668
- US-A- 4 570 026

## Beschreibung

Die vorliegende Erfindung betrifft ein Verfahren zur Herstellung von Isoolefinen, insbesondere Isobuten, durch Spaltung von Alkyl-tert.-alkylethern oder tertiären Alkoholen. Im Besonderen betrifft die vorliegende Erfindung ein Verfahren zur Spaltung von Alkyl-tert.-alkylethern, insbesondere MTBE, in Isoolefin und Alkohol.

Isoolefine, wie z. B. Isobuten, sind wichtige Zwischenprodukte für die Herstellung einer Vielzahl von organischen Verbindungen. Isobuten ist beispielsweise Ausgangsstoff für die Herstellung einer Vielzahl von Produkten, z. B. für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für die Herstellung von Methacrylsäure und deren Estern verwendet werden.

In technischen Strömen liegen Isoolefine meist zusammen mit anderen Olefinen und gesättigten Kohlenwasserstoffen mit gleicher oder unterschiedlicher Anzahl an Kohlenstoffatomen vor. Insbesondere aus Gemischen, die Isoolefine zusammen mit anderen Olefinen und gesättigten Kohlenwasserstoffen mit gleicher Anzahl an Kohlenstoffatomen pro Molekül aufweisen, sind die Isoolefine allein mit physikalischen Trennmethoden (wirtschaftlich) nicht abtrennbar. Beispielsweise liegt Isobuten in üblichen technischen Strömen zusammen mit gesättigten und ungesättigten C₄-Kohlenwasserstoffen vor. Aus diesen Gemischen kann Isobuten wegen der geringen Siedepunktsdifferenz bzw. des geringen Trennfaktors zwischen Isobuten und 1-Buten durch Destillation nicht wirtschaftlich abgetrennt werden.

Daher wird Isobuten aus technischen Kohlenwasserstoffgemischen üblicherweise dadurch gewonnen, dass Isobuten zu einem Derivat umgesetzt wird, das sich leicht vom übrig gebliebenen Kohlenwasserstoffgemisch abtrennen lässt, und dass das isolierte Derivat zu Isobuten und Derivatisierungsmittel zurückgespaltet wird.

Üblicherweise wird Isobuten aus C₄-Schnitten, beispielsweise der C₄-Fraktion eines Steamcrackers, wie folgt, abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt. Bei der Verwendung von Methanol entsteht aus Isobuten Methyl-tert.-butylether (MTBE) und bei Einsatz von Wasser tert.-Butanol (TBA). Nach ihrer Abtrennung können beide Produkte in Umkehrung ihrer Bildung zu Isobuten gespalten werden.

MTBE ist preiswerter als TBA, weil die Umsetzung von isobutenhaltigen Kohlenwasserstoffen mit Methanol leichter ist als mit Wasser und MTBE als Komponente von Ottokraftstoffen in großen Mengen produziert wird. Die Gewinnung von Isobuten aus MTBE ist daher potentiell wirtschaftlicher als die aus TBA, wenn für die Spaltung von MTBE ein ähnlich gutes Verfahren wie für die Spaltung von TBA zur Verfügung stünde.

Die Spaltung von Ethern mit einem tertiären Alkylrest zu den entsprechenden Isoolefinen und Alkoholen sowie die Spaltung von tertiären Alkoholen zu den entsprechenden Isoolefinen und Wasser kann in Gegenwart saurer Katalysatoren in der Flüssigphase bzw. Gas/Flüssig-Mischphase oder in der reinen Gasphase durchgeführt werden.

Die Spaltung in der Flüssigphase bzw. Gas/Flüssigphase hat den Nachteil, dass die entstehenden Produkte, gelöst in der Flüssigphase, Nebenreaktionen eingehen können. Beispielsweise bildet das bei der Spaltung von MTBE entstehende Isobuten durch sauer katalysierte Dimerisierung oder Oligomerisierung unerwünschte C₈- und C₁₂-Komponenten. Bei den unerwünschten C₈-Komponenten handelt es sich hauptsächlich um 2,4,4-Trimethyl-1-penten und 2,4,4-Trimethyl-2-penten. Darüber hinaus setzt sich ein Teil des bei der Spaltung entstehenden Methanols unter Wasserabspaltung zu Dimethylether um.

Mit steigender Spalttemperatur nehmen Nebenreaktionen, wie beispielsweise Hydrierungen oder Dehydrierungen zu. Darüber hinaus steigt mit zunehmender Temperatur der spezifische Energieverbrauch an. Weiterhin erfordern hohe Spalttemperaturen einen größeren Kapitaleinsatz für die Reaktoren. Es ist daher zweckmäßig, die Spaltung von Isoolefinderivaten bei Temperaturen unter 400 °C durchzuführen.

Es sind verschiedene Verfahren zur Herstellung von Isobuten durch Spaltung von MTBE in der Gasphase bekannt (US 4,570,026).

In DE 102 27 350 und DE 102 27 351 werden Verfahren zur Herstellung von Isobuten durch Spaltung von MTBE in der Gasphase beschrieben. In beiden Verfahren werden Temperaturen von 150 bis 300 °C eingesetzt. In US 6,072,095 und US 6,143,936 werden ebenfalls Verfahren zur Herstellung von Isobuten durch Spaltung von MTBE beschrieben. Bei diesen Verfahren werden Temperaturen von 50 bis 300 °C, vorzugsweise von 100 bis 250 °C eingesetzt. Problematisch bei der Durchführung der Spaltung in der Gasphase bei relativ tiefen Temperaturen ist eine schnellere Desaktivierung des Katalysators.

Da Katalysatoren während des Betriebs in ihrer Aktivität abnehmen, ist es vorteilhaft, um den Umsatz zu halten, durch Temperaturerhöhung gegenzusteuern. Um den Betrieb mit einem Katalysator möglichst lange aufrechterhalten zu können, ist deshalb eine möglichst tiefe Temperatur im vorgesehenen Temperaturbereich bei Einsatz vom frischen Katalysator wünschenswert.

Die Spaltung von Isoolefinderivaten ist endotherm. Bei der Spaltung der Isoolefinderivate kann deshalb in der ersten Katalysatorzone ein starker Temperaturabfall auftreten. Dies kann ausgehend von niedrigen Eingangstemperaturen dazu führen, dass die Temperatur im Katalysator besonders stark sinkt und der Katalysator dadurch schneller bzw. stärker desaktiviert wird.

Für die Durchführung von endothermen Reaktionen können Reaktoren eingesetzt werden, bei denen die Reaktionswärme von innen oder von außen zugeführt wird. Reaktoren mit innen liegender Beheizung (Heizstäbe oder Platten oder Röhren, die von einem Heizmedium durchströmt werden) erfordern wegen ihrer aufwändigen Konstruktion einen hohen Kapitaleinsatz. Bei Reaktoren, die von außen beheizt werden, handelt es sich meistens um einen Rohrreaktor bzw. Rohrbündelreaktor. Diese werden meistens mit Hilfe eines Wärmeträgers, das durch einen geschlossenen Mantel strömt, der das Rohr bzw. die Rohre umschließt, beheizt. Um bei endothermen Reaktionen den Temperaturabfall im Reaktor zu begrenzen, können anstelle eines Reaktors, mehrere in Reihe geschaltete Reaktoren, die mit unterschiedlichen Temperaturen betrieben werden, verwendet werden. Es kann auch ein Reaktor, dessen Mantel in unterschiedlichen Bereichen unterteilt ist, die mit Wärmeträger mit unterschiedlichen Temperaturen beschickt werden können, eingesetzt werden. Allerdings sind auch diese Konstruktionen aufwändig und erfordern einen hohen Kapitaleinsatz. Zudem sind die Betriebskosten höher als bei einer Umsetzung in einem Rohrreaktor mit nur einem Heizkreislauf.

Um den Temperaturabfall in der Katalysatorzone zu verringern, ist es auch möglich, in einem Rohrreaktor Katalysatoren unterschiedlicher Aktivität einzusetzen. So kann beispielsweise in einer ersten Katalysatorzone ein Katalysator mit einer geringeren Aktivität als in der folgenden Zone eingesetzt werden. Anstelle verschiedener Katalysatoren können auch Gemische eines Katalysators mit verschiedenen Anteilen eines Inertmaterials in den verschiedenen Zonen eingesetzt werden. Diese Vorgehensweisen haben den Nachteil, dass man verschiedene Katalysatoren bereithalten oder verschiedene Katalysatormischungen herstellen muss. Darüber hinaus ist die geschichtete Befüllung eines Rohrreaktors mit mehreren Katalysatoren oder Katalysatormischungen aufwändiger als die Befüllung mit einem Katalysator.

Es war daher die Aufgabe der vorliegenden Erfindung, ein alternatives Verfahren für die katalytische Gasphasenspaltung von Isoolefinderivaten zu Isoolefin sowie Alkohol oder Wasser in einer preiswerten und/oder einfachen Apparatur bereitzustellen, bei dem keine oder nur eine geringe Katalysatordesaktivierung auftritt.

Überraschenderweise wurde nun gefunden, dass Alkyl-tert.-alkylether und tertiäre Alkohole zu Isoolefinen mit 4 bis 6 C-Atomen sowie Alkohol oder Wasser an einem festen Katalysator in der Gasphase im Temperaturbereich 200 bis 400 °C bei einem Druck von 0,1 bis 1,2 MPa in einem einfachen Rohrreaktor bzw. Rohrbündelreaktor, der mit einem flüssigen Wärmeträger beheizt wird, auf einfache Weise gespaltet werden kann, ohne dass eine starke Katalysatordesaktivierung beobachtet wird, wenn der maximale Temperaturabfall an einer beliebigen Stelle in der Katalysatorzone auf kleiner 50 °C eingestellt wird, wenn das Reaktionsgemisch und der Wärmeträger im Gleichstrom (in getrennten Räumen) durch den Reaktor strömen und die Temperaturdifferenz des Wärmeträgers zwischen Zulaufstelle zum Reaktor und Ablauf aus dem Reaktor kleiner 40 °C beträgt.

Gegenstand der vorliegenden Erfindung ist daher ein kontinuierliches Verfahren zur Herstellung von Isoolefinen mit 4 bis 6 C-Atomen durch Spaltung von Verbindungen der Formel I

R₁-O-R₂ (I)

mit R₁ = einem 4 bis 6 Kohlenstoffatome aufweisenden tertiären Alkylrest und R₂ = H oder einem Alkylrest, in der Gasphase an einem festen Katalysator im Temperaturbereich 200 bis 400 °C bei einem Druck von 0,1 bis 1,2 MPa in einem Reaktor, der mit einem Heizmantel ausgestattet ist und der mit einem flüssigen Wärmeträger beheizt wird, welches dadurch gekennzeichnet ist, dass der Temperaturabfall in der Katalysatorzone an jeder beliebigen Stelle in Bezug auf die Eingangstemperatur kleiner 50 °C beträgt, dass das Reaktionsgemisch im Reaktor und der Wärmeträger im Mantel im Gleichstrom durch den Reaktor strömen und dass die Temperaturdifferenz des Wärmeträgers zwischen Zulaufstelle zum Reaktor und Ablauf aus dem Reaktor auf weniger als 40 °C eingestellt wird.

Das erfindungsgemäße Verfahren hat insbesondere den Vorteil, dass eine deutlich geringere Katalysatordesaktivierung beobachtet werden kann. Wird beispielsweise MTBE (Methyl-tert.-butylether), ETBE (Ethyl-tert.-butylether) oder TBA (tert.-Butanol) in Isobuten und Alkohol bzw. Wasser in der Gasphase gespaltet, so wird, um das entstehende Isobuten gegen Kühlwasser zu kondensieren, die Reaktion vorzugsweise bei erhöhtem Druck, beispielsweise 0,7 MPa, durchgeführt. Bei diesem Druck kann aber bereits bei Temperaturen unter 200 °C eine verstärkte Desaktivierung des Katalysators beobachtet werden. Für die Herstellung von Isoolefinen, insbesondere Isobuten, ist daher eine Spaltung bei einer Temperatur im Bereich von 200 °C bis 400 °C besonders vorteilhaft. Dies liegt möglicherweise daran, dass durch die erfindungsgemäße Verfahrensführung eine Kondensation von höher siedenden Komponenten auf dem Katalysator, die evtl. zur Katalysatordesaktivierung beiträgt, verringert oder vermieden werden kann.

Das erfindungsgemäße Verfahren weist weiter folgende Vorteile auf: Die Spaltung wird bevorzugt in einem Rohrbündelreaktor mit nur einem Heizkreislauf durchgeführt. Daraus ergeben sich im Vergleich zu aufwändiger konstruierten Reaktorsystemen ein geringerer Kapitaleinsatz und geringere Betriebskosten. Da der Reaktor nur mit einem Katalysator befüllt ist, kann ein Katalysatorwechsel schnell und kostengünstig durchgeführt werden. Weiterhin wird die Katalysatordesaktivierung verringert bzw. verlangsamt, was die Standzeit des Katalysators verlängert. Daraus resultiert eine Verringerung der Katalysatorkosten und der dadurch bedingten Produktionsausfälle durch Katalysatorwechsel.

Nachfolgend wird die Erfindung beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so soll die Offenbarung nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können, ohne dass diese aus Gründen der besseren Übersichtlichkeit explizit genannt worden sind.

Das erfindungsgemäße kontinuierliche Verfahren zur Herstellung von Isoolefinen mit 4 bis 6 C-Atomen durch Spaltung von Verbindungen der Formel I

R₁-O-R₂ (I)

mit R₁ = einem 4 bis 6 Kohlenstoffatome aufweisenden tertiären Alkylrest und R₂ = H oder einem Alkylrest, insbesondere einem 1 bis 3 Kohlenstoffatome aufweisenden Alkylrest, in der Gasphase an einem festen Katalysator im Temperaturbereich 200 bis 400 °C bei einem Druck von 0,1 bis 1,2 MPa, vorzugsweise von 0,5 bis 0,9 MPa und besonders bevorzugt von 0,7 bis 0,8 MPa in einem Reaktor, der mit einem Heizmantel ausgestattet ist und der mit einem flüssigen Wärmeträger beheizt wird, zeichnet sich dadurch aus, dass die Spaltung so durchgeführt wird, dass der Temperaturabfall in der Katalysatorzone/Reaktionszone an jeder beliebigen Stelle in Bezug auf die Eingangstemperatur kleiner 50 °C beträgt, dass das Reaktionsgemisch im Reaktor und der Wärmeträger im Mantel im Gleichstrom durch den Reaktor strömen und dass die Temperaturdifferenz des Wärmeträgers zwischen Zulaufstelle zum Reaktor und Ablauf aus dem Reaktor auf weniger als 40 °C eingestellt wird.

In dem erfindungsgemäßen Verfahren können als Verbindungen der Formel I z. B. tertiäre Alkohole mit 4 bis 6 C-Atomen eingesetzt werden. Insbesondere kann tert.-Butanol (TBA) zu Isobuten und Wasser gespalten werden. TBA kann aus verschiedenen technischen Prozessen stammen. Einer der wichtigsten ist die Umsetzung von Isobuten-haltigen C₄-Kohlenwasserstoffgemischen mit Wasser. Verfahren zur Herstellung von TBA sind beispielsweise in DE 103 30 710 und in US 7,002,050 beschrieben. TBA kann z. B. in reiner Form, als TBA/Wasser-Azeotrop oder als sonstiges TBA-Wasser-Gemisch eingesetzt werden.

In dem erfindungsgemäßen Verfahren können als Verbindungen der Formel I z. B. auch Alkyl-tert.-alkylether eingesetzt werden. Alkyl-tert.-alkylether, die nach dem erfindungsgemäßen Verfahren gespaltet werden können, sind beispielsweise MTBE, ETBE oder TAME (tert.-Amyl-methylether). Ein Verfahren zur Herstellung von MTBE ist beispielsweise in DE 101 02 062 beschrieben. Verfahren zur Herstellung von ETBE werden beispielsweise in DE 10 2005 062700, DE 10 2005 062722, DE 10 2005 062699 und DE 10 2006 003492 beschrieben.

Als Verbindungen der Formel I werden in dem erfindungsgemäßen Verfahren vorzugsweise tert.-Butanol, Methyl-tert.-butylether, Ethyl-tert.-butylether und/oder tert.-Amylmethylether eingesetzt. Es kann vorteilhaft sein, wenn ein Gemisch von zumindest zwei Verbindungen der Formel I eingesetzt wird. Dies kann z. B. dann der Fall sein, wenn bereits im Herstellprozess der zu spaltenden Verbindung der Formel I wegen der verwendeten Ausgangssubstanzen Gemische von Verbindungen der Formel I erhalten werden. Insbesondere wenn mit dem erfindungsgemäßen Verfahren Isobuten erzeugt werden soll, kann z. B. ein Gemisch, welches tert.-Butanol und Methyl-tert.-butylether aufweist, eingesetzt werden. Die Verbindungen der Formel I können als Reinstoff oder im Gemisch mit anderen Verbindungen dem erfindungsgemäßen Verfahren zu geführt werden. Insbesondere können dem erfindungsgemäßen Verfahren technische Mischungen, die eine oder mehrere Verbindungen der Formel I aufweisen zugeführt werden.

Als MTBE aufweisendes Gemisch kann im erfindungsgemäßen Verfahren MTBE unterschiedlicher Qualität eingesetzt werden. Als MTBE aufweisendes Gemisch kann/können z. B. reines MTBE, Gemische aus MTBE und Methanol, technisches MTBE verschiedener Qualitäten oder Gemische aus technischem MTBE und Methanol eingesetzt werden. Insbesondere können technisches MTBE verschiedener Qualitäten oder Gemische aus technischem MTBE und Methanol eingesetzt werden. Technisches MTBE (Kraftstoffqualität) ist, insbesondere aus wirtschaftlichen Gründen, der bevorzugte Einsatzstoff. Die Tabelle 1 zeigt beispielsweise die typische Zusammensetzung eines technischen MTBE der OXENO Olefinchemie GmbH.

**Tabelle 1: Typische Zusammensetzung von Technischem MTBE (Kraftstoffqualität) der Oxeno Olefinchemie GmbH.**

| Massenanteile [kg/kg] | |
|---|---|
| 1-Buten / 2-Butene | 0,001000 |
| Pentane | 0,001500 |
| MTBE | 0,978000 |
| 2-Methoxybutan | 0,003000 |
| Methanol | 0,008500 |
| tert.-Butanol | 0,003000 |
| Wasser | 0,000050 |
| Diisobuten | 0,003300 |

Technisches MTBE kann nach bekannten Verfahren durch Umsetzung von C₄-Kohlenwasserstoffgemischen, aus denen die mehrfach ungesättigten Kohlenwasserstoffe weitgehend entfernt worden sind, beispielsweise Raffinat I oder selektiv hydriertes Crack-C₄, mit Methanol hergestellt werden. Ein Verfahren zur Herstellung von MTBE wird beispielsweise in DE 101 02 062 beschrieben.

In dem erfindungsgemäßen Verfahren kann es besonders vorteilhaft sein, wenn ein MTBE aufweisender Strom eingesetzt wird, der ganz oder teilweise durch Abtrennen von Leichtsiedern in einem optionalen Verfahrensschritt aus einem MTBE aufweisenden Strom erhalten wird.

Das Abtrennen von Leichtsiedern kann insbesondere dann vorteilhaft sein, wenn der MTBE enthaltende Strom z. B. C₄- und/oder C₅-Kohlenwasserstoffe aufweist. Das Abtrennen der Leichtsieder, wie z. B. C₄- und/oder C₅-Kohlenwasserstoffe aus dem Strom in dem optionalen Verfahrensschritt kann vorzugsweise in einer Destillationskolonne erfolgen. Die Destillationskolonne wird vorzugsweise so betrieben, dass die Leichtsieder als Kopfprodukt abgetrennt werden können.

Vorzugsweise wird die Abtrennung der Leichtsieder in einer Destillationskolonne durchgeführt, die 30 bis 75 theoretische Trennstufen, bevorzugt 40 bis 65 und besonders bevorzugt 40 bis 55 theoretische Trennstufen aufweist. Vorzugsweise wird die Kolonne, in Abhängigkeit von der realisierten Stufenzahl, der Zusammensetzung des eingesetzten MTBE und der erforderlichen Reinheit an C₄- und C₅-Kohlenwasserstoffen, mit einem Rücklaufverhältnis zwischen 150 und 350, insbesondere zwischen 200 und 300 betrieben. Die Kolonne im optionalen Verfahrenschritt wird vorzugsweise mit einem Betriebsdruck von 0,2 bis 0,6 MPa_{(abs)}, bevorzugt von 0,3 bis 0,4 MPa_{(abs)} betrieben. Zur Beheizung der Kolonne kann z. B. 0,4-MPa Dampf eingesetzt werden. Die Kondensation kann, je nach gewähltem Betriebsdruck, gegen Kühlsohle, Kühlwasser oder Luft erfolgen. Der Kopfbrüden der Kolonne können vollständig oder nur teilweise kondensiert werden, so dass das Kopfprodukt entweder flüssig oder dampfförmig abgezogen werden kann. Das Kopfprodukt kann thermisch verwertet werden oder als Einsatzstoff einer Synthesegasanlage genutzt werden. Das Sumpfprodukt kann direkt der Spaltung zugeführt werden.

Das erfindungsgemäße Verfahren wird derart durchgeführt, dass an keiner Stelle der Katalysatorzone die Temperatur unter 200 °C fällt. Vorzugsweise liegt die Eingangstemperatur des gasförmigen Edukts deshalb über 200 °C, bevorzugt deutlich über 200 °C. Die Eingangstemperatur des Edukts kann in einem dem Reaktor vorgeschalteten Aufheizer eingestellt werden. Wird als Edukt ein MTBE als Verbindung der Formel I aufweisendes Edukt eingesetzt beträgt die Eingangstemperatur vorzugsweise mindestens 230 °C, bevorzugt größer 250 °C.

Bei Verwendung von frischem Katalysator, insbesondere bei der Verwendung von frischem Magnesiumoxid/Aluminiumoxid/Siliziumoxid-Katalysator bei der MTBE-Spaltung liegt die Eingangstemperatur bevorzugt zwischen 250 und 270 °C. Im Laufe des Betriebes kann es vorteilhaft sein, mit zunehmender Desaktivierung des Katalysators zur Konstanthaltung des Umsatzes die Eingangstemperatur bis auf 400 °C anzuheben. Kann der Umsatz bei Erreichen von 400 °C nicht mehr gehalten werden, so kann es vorteilhaft sein, den Katalysator ganz oder teilweise zu ersetzen.

Der Temperaturabfall in der Katalysatorzone beträgt an jeder beliebigen Stelle in Bezug auf die Eingangstemperatur kleiner als 50 °C, vorzugsweise kleiner als 40 °C und besonders bevorzugt 1 bis 30 °C. Der maximale Temperaturabfall kann durch zahlreiche Parameter, wie z. B. durch die Temperatur des zum Heizen verwendeten Wärmeträgers sowie durch die Geschwindigkeit, mit der der Wärmträger durch den Mantel strömt, eingestellt werden.

Der Reaktor wird vorzugsweise mit einer Raumgeschwindigkeit (Weight Hourly Space Velocity (WHSV) in Kilogramm Edukt je Kilogramm Katalysator je Stunde) von 0,1 bis 5 h⁻¹, insbesondere von 1 bis 3 h⁻¹ im geraden Durchgang betrieben.

Der Reaktor kann in jeder beliebigen Raumrichtung angeordnet sein. Weist der Reaktor Reaktorrohre auf, so können diese ebenfalls in jede beliebige Raumrichtung weisen. Bevorzugt ist der Reaktor jedoch derart aufgestellt, dass der Reaktor bzw. die Reaktorrohre vertikal ausgerichtet sind. Bei einem senkrecht ausgerichteten Reaktor wird der Wärmeträger bevorzugt an der höchsten Stelle oder in der Nähe der höchsten Stelle des Mantels zugeführt und an der tiefsten Stelle oder in der Nähe der tiefsten Stelle des Reaktors abgezogen oder umgekehrt. Das Reaktionsgemisch in der Reaktionszone und der Wärmeträger im Mantel durchströmen den Reaktor in gleicher Richtung. Besonders bevorzugt durchströmen der Wärmeträger und das Reaktionsgemisch den Mantel des Reaktors bzw. die Reaktionszone des Reaktors von oben nach unten.

Um eine gleichmäßigere Beheizung der Reaktionszone zu erreichen, kann es vorteilhaft sein, den Wärmeträger nicht nur an einer Stelle, sondern an mehreren Stellen in etwa gleicher Höhe in den Reaktor einzuspeisen. Um bei der Verwendung eines Rohrbündelreaktors einen größeren Temperaturabfall in den mittleren Rohren im Vergleich zu Randrohren zu vermeiden, kann es vorteilhaft sein, in dem Zulauf oder in den Zuläufen für den Wärmeträger Düsen vorzusehen, die den Transport des Wärmeträgers zu den mittleren Rohren begünstigen. Auf diese Weise können Temperaturschwankungen über den Querschnitt des Rohrbündels vermieden werden.

Der Wärmeträger kann an einer oder mehreren Stelle(n) den Reaktor verlassen. Wird der Reaktor vom Wärmeträger von oben nach unten durchströmt, ist durch konstruktive Maßnahmen sicherzustellen, dass die Reaktionszonen, wie z. B. die Reaktionsrohre, vollständig mit Wärmeträger umspült werden.

Der Wärmeträger kann außerhalb des Reaktors durch direkte oder indirekte Beheizung auf die gewünschte Temperatur gebracht und durch den Reaktor gepumpt werden.

Als Wärmeträger können Salzschmelzen, Wasser oder Wärmeträgeröle verwendet werden. Für den Temperaturbereich von 200 bis 400 °C ist die Verwendung von Wärmeträgerölen vorteilhaft, da Heizkreisläufe mit ihnen im Vergleich zu anderen technischen Lösungen einen geringeren Kapitaleinsatz erfordern. Wärmeträgeröle, die eingesetzt werden können, sind beispielsweise solche, die unter den Handelsnamen Marlotherm (z. B. Marlotherm SH der Sasol Olefins & Surfactants GmbH), Diphyl (Fa. Bayer), Dowtherm (Fa. Dow) oder Therminol (Fa. Therminol) vertrieben werden. Diese synthetisch hergestellten Wärmeträgeröle basieren im Wesentlichen auf thermisch stabilen Ringkohlenwasserstoffen.

Vorzugsweise wird der Wärmeträger mit einer Temperatur, die 10 bis 40 °C, bevorzugt 10 bis 30 °C höher ist als die Temperatur des in den Reaktor strömenden Edukts, in den Heizmantel des Reaktors geleitet. Die Temperaturdifferenz des flüssigen Wärmeträgers über den Reaktor, also zwischen Eintrittstemperatur des Wärmeträgers bei Eintritt in den Heizmantel und der Austrittstemperatur des Wärmeträgers bei Austritt aus dem Heizmantel beträgt vorzugsweise weniger als 40 °C, bevorzugt weniger als 30 °C und besonders bevorzugt 10 bis 25 °C. Die Temperaturdifferenz kann durch den Massenfluss des Wärmeträgers pro Zeiteinheit (Kilogramm je Stunde) durch den Heizmantel eingestellt werden.

Im erfindungsgemäßen Verfahren können alle festen Katalysatoren eingesetzt werden, die im Temperaturbereich von 200 bis 400 °C die Spaltung von tert.-Alkoholen und Alkyl-tert.-Alkylethern zu Isoolefinen ermöglichen. Vorzugsweise werden Katalysatoren eingesetzt, mit denen im Reaktor eine Reaktionsgeschwindigkeit von höchstens 400 mol/(h·kg_{KAT}), bevorzugt zwischen 1 und 400 mol/(h·kg_{KAT}) erzielt wird.

Die im erfindungsgemäßen Verfahren eingesetzten Katalysatoren können z. B. Metalloxide, Metallmischoxide, insbesondere solche, die Siliziumoxid und/oder Aluminiumoxid enthalten, Säuren auf Metalloxidträgern oder Metallsalze oder Mischungen davon sein.

Im erfindungsgemäßen Verfahren werden zur Spaltung von MTBE zu Isobuten und Methanol in der Gasphase bevorzugt Katalysatoren verwendet, die formal aus Magnesiumoxid, Aluminiumoxid und Siliziumoxid bestehen. Solche Katalysatoren werden z. B. in US 5,171,920 in Beispiel 4 und in EP 0 589 557 beschrieben.

Besonders bevorzugt werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Dieses kann z. B. ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der erfindungsgemäße Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g. Das Porenvolumen wird vorzugsweise bestimmt durch die Cyclohexanmethode. Bei dieser Methode wird die zu prüfende Probe zunächst bei 110 °C bis zur Gewichtskonstanz getrocknet. Anschließend werden ca. 50 ml der auf 0,01 g genau eingewogenen Probe in ein gereinigtes und bis zur Gewichtskonstanz getrocknetes Imprägnierrohr gefüllt, dass an der Unterseite eine Auslauföffnung mit einem Schliffhahn aufweist. Die Auslauföffnung ist mit einem kleinen Plättchen aus Polyethylen abgedeckt, wodurch ein Verstopfen der Auslauföffnung durch die Probe verhindert wird. Nach dem Befüllen des Imprägnierrohres mit der Probe wird das Rohr sorgfältig luftdicht verschlossen. Anschließend wird das Imprägnierrohr mit einer Wasserstrahlpumpe verbunden, der Schliffhahn geöffnet und über den Wasserstrahl ein Vakuum im Imprägnierrohr von 20 mbar eingestellt. Das Vakuum kann an einem parallel angeschlossenen Vakuummeter geprüft werden. Nach 20 min. wird Schliffhahn geschlossen und das evakuierte Imprägnierrohr wird anschließend so mit einer Cyclohexanvorlage, in der ein genau abgemessenes Volumen an Cyclohexan vorgelegt wird, verbunden, dass durch Öffnen des Schliffhahns Cyclohexan aus der Vorlage in das Imprägnierrohr gesaugt wird. Der Schliffhahn bleibt so lange geöffnet, bis die gesamte Probe mit Cyclohexan geflutet ist. Anschließend wird der Schliffhahn wieder verschlossen. Nach 15 min wird das Imprägnierrohr vorsichtig belüftet und das nicht absorbierte Cyclohexan in die Vorlage abgelassen. Im Imprägnierrohr bzw. in der Auslauföffnung oder der Verbindung mit der Cyclohexanvorlage anhaftendes Cyclohexan kann durch einen einzelnen vorsichtigen Druckstoß aus einem Saugball, über die Belüftungsleitung in die Vorlage befördert werden. Das Volumen des in der Vorlage vorhandenen Cyclohexans wird notiert. Das Porenvolumen ergibt sich aus dem absorbierten Cyclohexanvolumen, welches bestimmt wird aus dem Cyclohexanvolumen in der Vorlage vor der Messung minus dem Cyclohexanvolumen in der Vorlage nach der Messung, geteilt durch die Masse der untersuchten Probe.

Der mittlere Porendurchmesser (vorzugsweise bestimmt in Anlehnung an DIN 66133) des Katalysators beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer-gleich 3,5 nm bestimmt mit Quecksilberporosimetrie gemäß DIN 66133) des Katalysators auf Poren mit einem Durchmesser von 3,5 bis 50 nm (Mesoporen).

Im erfindungsgemäßen Verfahren werden bevorzugt Katalysatoren eingesetzt, die eine mittlere Korngröße (bestimmt durch Siebanalyse) von 10 µm bis 10 mm, vorzugsweise 0,5 mm bis 10 mm, besonders bevorzugt eine mittlere Korngröße von 1 bis 5 mm aufweisen. Vorzugsweise werden feste Katalysatoren eingesetzt, die eine mittlere Korngröße d₅₀, von 2 bis 4 mm, insbesondere von 3 bis 4 mm.

In dem erfindungsgemäßen Verfahren kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Der Katalysator kann auch aufgebracht sein auf einem Träger, wie z. B. einen Metall-, Kunststoff- oder Keramikträger, vorzugsweise auf einem in Bezug auf die Reaktion, bei welcher der Katalysator eingesetzt werden soll, inerten Träger. Insbesondere kann der erfindungsgemäße Katalysator auf einen Metallträger, wie z. B. eine Metallplatte oder eine Metallgewebe aufgebracht sein. Solche mit dem erfindungsgemäßen Katalysator ausgerüstete Träger können z. B. als Einbauten in Reaktoren oder Reaktivdestillationskolonnen verwendet werden. Die Träger können auch Metall-, Glas- oder Keramikkugeln oder Kugeln von anorganischen Oxiden sein. Ist der erfindungsgemäße Katalysator auf einem inerten Träger aufgebracht, so wird die Masse und Zusammensetzung des inerten Trägers bei der Bestimmung der Zusammensetzung des Katalysators nicht berücksichtigt.

Die besonders bevorzugt einzusetzenden, formal Magnesiumoxid (MgO), Aluminiumoxid (Al₂O₃) und Siliziumdioxid (SiO₂) aufweisenden Katalysatoren können z. B. mit einem Verfahren hergestellt werden, das die Schritte
a) Behandeln eines Alumosilikats mit einer sauren, wässrigen Magnesiumsalzlösung und
b) Kalzinieren des mit wässriger Magnesiumsalzlösung behandelten Alumosilikats,
aufweist. Unter Alumosilikaten sollen dabei Verbindungen verstanden werden, die sich im Wesentlichen formal aus Anteilen von Aluminiumoxid (Al₂O₃) und Siliziumdioxid (SiO₂) zusammensetzen. Die Alumosilikate können aber auch noch geringe Anteile an Alkali- oder Erdalkalimetalloxiden enthalten. In dem Verfahren können als Alumosilikate auch Zeolithe wie z. B. Zeolith A, X, Y, USY oder ZSM-5 oder amorphe Zeolithe (wie beispielsweise MCM 41 von Mobil Oil) eingesetzt werden. Die im Verfahren eingesetzten Alumosilikate können amorph oder kristallin sein. Geeignete kommerzielle Alumosilikate die als Ausgangsstoffe im erfindungsgemäßen Verfahren eingesetzt werden können sind beispielsweise Alumosilikate, die durch Fällung, Gelierung oder Pyrolyse hergestellt wurden. In dem Verfahren werden vorzugsweise Alumosilikate eingesetzt, die von 5 bis 40 Massen-%, bevorzugt 10 bis 35 Massen-% Aluminiumoxid und von 60 bis 95 Massen-%, bevorzugt von 65 bis 90 Massen-% Siliziumdioxid aufweisen (bezogen auf die Trockenmasse; Behandlung: Glühen bei 850 °C für 1 h). Die Bestimmung der Zusammensetzung der eingesetzten Alumosilikate bzw. der erhaltenen Katalysatoren kann z. B. durch klassische Analyse, Schmelzaufschluss mit Borax und RFA (Röntgenfluoreszenzanalyse), energiedispersive Röntgenanalyse, Flammenspektroskopie (Al und Mg, nicht Si), nassem Aufschluss und anschließender ICP-OES (Optische Emissionsspektrometrie mit induktiv gekoppelten Hochfrequenzplasma) oder Atomabsorptionsspektroskopie erfolgen. Ein besonders bevorzugtes Alumosilikat, welches in dem Verfahren eingesetzt werden kann, weist einen formalen Anteil an Al₂O₃ von 13 Massen-% und einen Anteil an Siliziumdioxid von 76 Massen-% auf. Ein solches Alumosilikat wird von der Firma Grace Davison, unter der Bezeichnung Davicat O 701 angeboten.

Das Alumosilikat kann in den unterschiedlichsten Formen in dem Verfahren eingesetzt werden. So kann das Alumosilikat in Form von Formkörpern, wie z. B. Tabletten, Pellets, Granulat, Strängen oder Extrudaten eingesetzt werden. Das Alumosilikat kann aber auch als Alumosilikatpulver eingesetzt werden. Als Ausgangsmaterial kann von Pulvern mit unterschiedlicher mittlerer Korngröße und unterschiedlicher Korngrößenverteilung ausgegangen werden. Bevorzugt wird in dem erfindungsgemäßen Verfahren ein Alumosilikatpulver eingesetzt, bei dem 95 % der Partikel eine mittlere Korngröße von 5 bis 100 µm, vorzugsweise 10 bis 30 µm und besonders bevorzugt 20 bis 30 µm aufweisen. Die Bestimmung der Korngröße kann z. B. durch Laserbeugung mit einem Partikelanalysator der Firma Malvern, wie z. B. dem Mastersizer 2000 durchgeführt werden.

Zur Herstellung der wässrigen Magnesiumsalzlösung werden Magnesiumverbindungen verwendet werden, die wasserlöslich sind oder durch Zugabe einer Säure in wasserlösliche Verbindungen übergehen. Vorzugsweise werden als Salze die Nitrate eingesetzt. Bevorzugt werden Magnesiumsalzlösungen eingesetzt, die als Magnesiumsalze die Salze starker Mineralsäuren, wie beispielsweise Magnesiumnitrat-Hexahydrat oder Magnesiumsulfat-Heptahydrat aufweisen. Die eingesetzte saure wässrige Alkali- und/oder Erdalkalimetallsalzlösung weist vorzugsweise einen pH-Wert von kleiner 6, bevorzugt von kleiner 6 bis 3, und besonders bevorzugt von 5,5 bis 3,5 auf. Die Bestimmung des pH-Werts kann z. B. mit Hilfe einer Glaselektrode oder Indikatorpapier durchgeführt werden. Weist die Salzlösung einen pH-Wert auf, der größer oder gleich 6 ist, so kann der pH-Wert durch Zugabe einer Säure, vorzugsweise der Säure dessen Alkali- und/oder Erdalkalimetallsalz in der Lösung vorhanden ist, eingestellt werden. Vorzugsweise wird, wenn die Alkali- und/oder Erdalkalimetallsalzlösung als Salze die Nitrate aufweist, als Säure Salpetersäure eingesetzt. Der Magnesiumgehalt der eingesetzten Magnesiumsalzlösung beträgt vorzugsweise von 0,1 bis 3 Mol/l, bevorzugt von 0,5 bis 2,5 Mol/l.

Das Behandeln in Schritt a) kann auf verschiedene Weisen erfolgen, die geeignet sind, das Alumosilikat mit der Magnesiumsalzlösung in Kontakt zu bringen. Mögliche Behandlungsmethoden sind z. B. Imprägnieren, Tränken, Besprühen oder Begießen des Alumosilikats mit der Magnesiumsalzlösung. Es kann vorteilhaft sein, wenn das Behandeln des Alumosilikats so erfolgt, dass die Magnesiumsalzlösung zumindest 0,1 bis 5 h bevorzugt von 0,5 bis 2 h auf das Alumosilikat einwirken kann. Eine solche Einwirkzeit kann insbesondere dann vorteilhaft sein, wenn das Behandeln durch einfaches Tränken erfolgt.

In einer bevorzugten Ausführungsform des erfindungsgemäßen Schrittes a) des erfindungsgemäßen Verfahrens kann die Behandlung von Alumosilikat, insbesondere Alumosilikatformkörpern mit der Magnesiumsalzlösung beispielsweise durch Vakuumimprägnierung in einer dafür geeigneten Vakuumimprägnieranlage erfolgen. Bei dieser Art der Behandlung wird das Alumosilikat in der Vakuumimprägnieranlage zunächst evakuiert. Anschließend wird die Magnesiumsalzlösung bis über den oberen Rand der Trägerschüttung gesaugt, so dass das gesamte Alumosilikat mit der Lösung bedeckt ist. Nach einer Einwirkzeit, die vorzugsweise von 0,1 bis 10 h, bevorzugt von 0,5 bis 2 h beträgt, wird die Lösung, die nicht vom Träger aufgenommen wurde, abgelassen.

In einer weiteren bevorzugten Ausführungsform des erfindungsgemäßen Schrittes a) des erfindungsgemäßen Verfahrens kann die Behandlung von Alumosilikat, insbesondere Alumosilikatformkörpern mit der Alkali- und/oder Erdalkalimetallsalzlösung beispielsweise durch Besprühen oder Begießen des Alumosilikats erfolgen. Vorzugsweise erfolgt das Besprühen oder Begießen des Alumosilikats mit der Magnesiumsalzlösung in dem die Lösung auf das in einer Trommel rotierende Alumosilikat gesprüht oder gegossen wird. Die Behandlung kann in einem Zug erfolgen, d. h. zum Alumosilikat wird zu Beginn die gesamte Menge an Magnesiumsalzlösung in einem Schritt zugegeben. Die Salzlösung kann aber auch durch Besprühen oder Begießen in kleinen Portionen zudosiert werden, wobei der Zeitraum der Zugabe vorzugsweise von 0,1 bis 10 h und bevorzugt von 1 bis 3 h beträgt. Die Menge an Salzlösung wird vorzugsweise so bemessen, dass die gesamte Lösung vom Alumosilikat aufgenommen wird. Insbesondere das Tränken aber auch das Besprühen bzw. Begießen kann in üblichen technischen Apparaturen, wie beispielsweise Konusmischern oder Intensivmischern, wie sie z. B. von der Firma Eirich angeboten werden, durchgeführt werden.

Die Behandlung des Alumosilikats mit der Magnesiumsalzlösung in Schritt a) kann in einem Schritt oder in mehreren Teilschritten erfolgen. Insbesondere ist es möglich, die Behandlung in zwei oder mehreren Teilschritten durchzuführen. In jedem der einzelnen Teilschritte kann jeweils die gleiche Magnesiumsalzlösung eingesetzt werden oder aber in jedem Teilschritt eine in der Konzentration verschiedene Magnesiumsalzlösung. Beispielsweise kann zum Alumosilikat zunächst nur ein Teil der Magnesiumsalzlösung zugesetzt werden und, gegebenenfalls nach einer Zwischentrocknung, bei gleicher oder anderer Temperatur die Restmenge der eingesetzten Magnesiumsalzlösung. Es ist nicht nur möglich, dass der Schritt a) in zwei oder mehreren Teilschritten durchgeführt wird. Ebenfalls ist es möglich, dass das Verfahren mehrere Schritte a) aufweist. Auch in diesem Fall können in den verschiedenen Schritten a) gleiche oder unterschiedliche Magnesiumsalzlösung in Bezug auf die Konzentration eingesetzt werden.

Das Behandeln in Schritt a) kann vorzugsweise bei einer Temperatur von 10 bis 120 °C, bevorzugt von 10 bis 90 °C, besonders bevorzugt von 15 bis 60 °C und ganz besonders bevorzugt bei einer Temperatur von 20 bis 40 °C durchgeführt werden.

Es kann vorteilhaft sein, wenn in Schritt a) ein oder mehrere Additive dem Alumosilikat bzw. der Magnesiumsalzlösung zugegeben bzw. zugemischt werden. Solche Additive können z. B. Bindemittel, Gleitmittel oder Formgebungshilfsmittel sein. Ein geeignetes Bindemittel kann z. B. Boehmit oder Pesudo-Boehmit sein, wie es z. B. unter der Bezeichnung Disperal (einem Boehmit mit einem formalen Al₂O₃-Gehalt von ca. 77 Massen-%) von der Sasol Deutschland GmbH angeboten wird. Wird Boehmit, insbesondere Disperal als Bindemittel zugegeben, so wird dieses vorzugsweise als Gel zugegeben, welches z. B. durch Einrühren von 197 Massenteilen Disperal in 803 Massenteilen einer 1,28 Massen-%igen, wässrigen Salpetersäure, gründliches Rühren bei 60 °C für 3 h, Abkühlen auf Raumtemperatur und Ergänzen von evtl. verdunstetem Wasser erhalten werden kann. Als Formgebungshilfsmittel können beispielsweise Kieselsäuren, insbesondere pyrogene Kieselsäuren, wie sie z. B. von der Degussa AG unter der Bezeichnung Aerosil vertrieben werden, Bentonite, Tone, Kaolin, Kaolinit, ball clay und andere, dem Fachmann hierfür geläufige Stoffe eingesetzt werden. Als Gleitmittel, dessen Einsatz für die verbesserte Tablettierung vorteilhaft sein kann, kann beispielsweise Graphit zugesetzt werden.

Die Zugabe eines oder mehrerer der oben genannten Additive in Schritt a) kann auf verschiedene Weisen erfolgen. Insbesondere kann die Zugabe während der Behandlung des Alumosilikats mit der Magnesiumsalzlösung erfolgen. Beispielweise können Alumosilikat, Additiv und Magnesiumsalzlösung in eine technische Apparatur gefüllt und anschließend innig vermischt werden. Eine andere Möglichkeit besteht darin, zuerst das Alumosilikat mit dem Additiv zu vermischen und anschließend die Magnesiumsalzlösung zuzusetzen. In einer weiteren Variante kann zum Alumosilikat Additiv und Magnesiumsalzlösung gleichzeitig zudosiert werden. Die Zugabe kann jeweils in einem Guss, in Portionen oder durch Sprühen erfolgen. Die Zugabezeit beträgt vorzugsweise weniger als 5 h, bevorzugt weniger als 3 h. Es kann vorteilhaft sein, die Mischung für 0,1 bis 10 h, bevorzugt für 0,5 bis 3 h, nachzumischen.

Das Herstellverfahren für den bevorzugt eingesetzten Katalysator weist zumindest einen Verfahrensschritt b) auf, bei dem das mit Alkali- und/oder Erdalkalimetallsalzlösung behandelte Alumosilikat kalziniert wird. Die Kalzinierung erfolgt vorzugsweise in einem Gasstrom, beispielsweise in einem Gasstrom, der z. B. Luft, Stickstoff, Kohlendioxid und/oder eines oder mehrere Edelgase enthält oder aus einer oder mehrerer dieser Komponenten besteht. Bevorzugt erfolgt die Kalzinierung unter Verwendung von Luft als Gasstrom.

Die Kalzinierung in Verfahrensschritt b) wird vorzugsweise bei einer Temperatur von 200 bis 1000 °C, bevorzugt von 300 bis 800 °C durchgeführt. Die Kalzinierung erfolgt vorzugsweise für eine Dauer von 0,1 bis 10 Stunden, bevorzugt von 1 bis 5 Stunden. Besonders bevorzugt wird die Kalzinierung bei einer Temperatur von 200 bis 1000 °C, vorzugsweise 300 bis 800 °C für 0,1 bis 10 Stunden, bevorzugt von 1 bis 5 Stunden durchgeführt.

Bevorzugt kann die technische Kalzinierung in einem Schachtofen durchgeführt werden. Die Kalzinierung kann jedoch auch in anderen bekannten technischen Apparaturen durchgeführt werden, wie beispielsweise Wirbelschichtkalzinierern, Drehrohröfen oder Hordenöfen.

Es kann vorteilhaft sein, wenn zwischen den Schritten a) und b) ein Schritt c) durchgeführt wird, in dem das mit Magnesiumsalzlösung behandelte Alumosilikat getrocknet wird. Die Trocknung in Schritt c) kann bei einer Temperatur von 100 bis 140 °C erfolgt. Vorzugsweise erfolgt die Trocknung in einem Gasstrom. Die Trocknung kann beispielsweise in einem Gasstrom, der z. B. Luft, Stickstoff, Kohlendioxid und/oder eines oder mehrere Edelgase enthält oder aus einer oder mehrerer dieser Komponenten besteht, durchgeführt werden. Durch den Zwischenschritt des Trocknens nach der Behandlung mit Alkali- und/oder Erdalkalimetallsalzlösung und vor dem Kalzinieren kann erreicht werden, dass bei der Kalzinierung keine großen Wasserdampfmengen freigesetzt werden. Zudem kann durch das Trocknen verhindert werden, dass durch beim Kalzinieren spontan verdampfendes Wasser die Form des Katalysators zerstört.

Je nachdem in welcher gewünschten Form der Katalysator vorliegen soll, kann es vorteilhaft sein, das Herstellungsverfahren durch zusätzliche Verfahrensschritte entsprechend anzupassen. Soll mit dem Verfahren beispielsweise pulverförmiger Katalysator hergestellt werden, so kann das Alumosilikat in Form von Alumosilikatpulver eingesetzt und z. B. in einem Konusmischer mit der Magnesiumsalzlösung behandelt (z. B. durch Imprägnieren) optional getrocknet und anschließend kalziniert werden. Ein pulverförmiger Katalysator kann aber auch dadurch hergestellt werden, dass ein Katalysatorformkörper durch Mahlen und Sieben zu pulverförmigem Katalysator verarbeitet wird.

Die Katalysatorformkörper können beispielsweise in Form von Strängen, Kugeln, Pellets oder Tabletten vorliegen. Um zum formgegebenen Katalysator (Katalysatorformkörper) zu gelangen, können abhängig von der jeweiligen Formgebungsvariante neben den Verfahrensschritten Behandlung, Trocknung, Kalzination, weitere Verfahrensschritte, wie z. B. Formgebung, Mahlung oder Siebung, durchgeführt werden. Formgebungshilfsmittel können an verschiedenen Stellen im Prozess eingebracht werden. Die Herstellung der Katalysatorformkörper kann auf unterschiedliche Weise erfolgen:

In einer ersten Ausführungsvariante können Katalysatorformkörper, insbesondere erfindungsgemäße Katalysatorformkörper, dadurch erhalten werden, dass Alumosilikatformkörper mit einer sauren wässrigen Magnesiumsalzlösung behandelt, optional getrocknet und anschließend kalziniert werden.

In einer zweiten Ausführungsform kann ein Katalysatorformkörper dadurch erhalten werden, dass ein Alumosilikatpulver, zunächst mit einer sauren wässrigen Magnesiumsalzlösung behandelt wird, dann gegebenenfalls getrocknet und anschließend kalziniert wird, und anschließend das erhaltene Katalysatorpulver durch in der Technik übliche Verfahren wie beispielsweise Kompaktierung, Extrusion, Pelletierung, Tablettierung, Granulation oder Coating zu Katalysatorformkörpern verarbeitet wird. Für die Formgebung benötigte Additive, wie z. B. Bindemittel oder weitere Hilfsstoffe können dabei an verschiedenen Stellen im Herstellungsprozess, so z. B. im Verfahrensschritt a) zugegeben werden. Beim Herstellen eines Formkörpers aus einem Alumosilikatpulver als Ausgangsmaterial kann von Pulvern mit unterschiedlicher mittlerer Korngröße und unterschiedlicher Korngrößenverteilung ausgegangen werden. Bevorzugt wird für die Herstellung von Formkörpern ein Alumosilikatpulver eingesetzt, bei dem 95 % der Partikel eine Korngröße von 5 bis 100 µm, vorzugsweise von 10 bis 30 µm und besonders bevorzugt von 20 bis 30 µm aufweisen (Bestimmt durch Laserbeugung, siehe oben).

In einer dritten Ausführungsform des Verfahrens können Pellets des Katalysators dadurch erhalten werden, dass in Verfahrensschritt a) ein Alumosilikatpulver mit einer sauren wässrigen Magnesiumsalzlösung behandelt, optional getrocknet (Verfahrensschritt c)) und anschließend in Verfahrenschritt b) kalziniert wird und das so erhaltene Katalysatorpulver, z. B. in einem Eirichmischer, unter Zugabe von Bindemittel pelletiert wird und die erhaltenen Pellets in einem weiteren Verfahrensschritt c) getrocknet und anschließend in einem weiteren Verfahrensschritt b) kalziniert werden.

In einer vierten Ausführungsform des Herstellverfahrens können Pellets des Katalysators dadurch erhalten werden, dass in Verfahrensschritt a) ein Alumosilikatpulver, Bindemittel und saure wässrige Magnesiumsalzlösung gemischt werden und das so behandelte Alumosilikatpulver, z. B. in einem Eirichmischer, pelletiert wird und die erhaltenen feuchten Pellets in Verfahrensschritt c) getrocknet und anschließend in Verfahrensschritt b) im Gasstrom kalziniert werden.

In einer fünften Ausführungsform des Herstellverfahrens können Tabletten des Katalysators dadurch erhalten werden, dass in Verfahrensschritt a) ein Alumosilikatpulver, Bindemittel, optional Gleitmittel und saure wässrige Magnesiumsalzlösung gemischt werden und das so behandelte Alumosilikatpulver, z. B. in einem Eirichmischer, zu Mikropellets vorzugsweise mit einem mittleren Durchmesser 0,5 bis 10 mm, bevorzugt 1 bis 5 mm und besonders bevorzugt von 1 bis 3 mm (Bestimmung der Korngröße kann z. B. durch Siebanalyse erfolgen) pelletiert wird und die erhaltenen feuchten Pellets in Verfahrensschritt c) getrocknet und anschließend optional in Verfahrensschritt b) im Gasstrom kalziniert werden. Die erhaltenen Pellets können dann, falls in Verfahrensschritt a) noch nicht geschehen mit einem Gleitmittel, wie z. B. Graphit, vermischt und anschließend auf einer handelsüblichen Tablettenpresse, z. B. einer Rundläuferpresse, tablettiert werden. Die Tabletten können dann, falls noch kein Verfahrensschritt b) durchgeführt wurde, in Verfahrensschritt b) kalziniert werden oder optional nachkalziniert werden.

In einer sechsten Ausführungsform des Herstellverfahrens können Tabletten des Katalysators dadurch erhalten werden, dass vorgeformte Formkörperkatalysatoren, wie sie z. B. als Pellets in Ausführungsform drei oder vier erhaltenen werden können, gemahlen und das erhaltene Granulat/Pulver gesiebt wird, so dass ein tablettierfähiges Granulat von Katalysator erhalten wird, und diesem Granulat Gleitmittel zugemischt wird. Das so vorbereitete Granulat kann anschließend tablettiert werden. Die Tabletten können dann, falls noch kein Verfahrensschritt b) durchgeführt wurde, in Verfahrensschritt b) kalziniert werden. Das Zumischen eines Gleitmittels kann entfallen, wenn bereits bei der Herstellung der Pellets, z. B. in Verfahrenschritt a) ein Gleitmittel zugefügt wurde.

In einer siebten Ausführungsform des erfindungsgemäßen Verfahrens können mit dem Katalysator beschichtete Materialien/Träger hergestellt werden. Bei dieser Ausführungsform wird zunächst ein Katalysatorpulver dadurch hergestellt werden, dass in Verfahrensschritt a) ein Alumosilikatpulver mit einer sauren wässrigen Magnesiumsalzlösung behandelt, optional getrocknet (Verfahrensschritt c)) und optional kalziniert (Verfahrenschritt b)) wird. Das so erhaltene Katalysatorpulver, wird anschließend in einem Suspensionsmittel, wie z. B. Wasser oder Alkohol suspendiert, wobei gegebenenfalls ein Bindemittel der Suspension zugegeben werden kann. Die so hergestellte Suspension kann dann auf jedes beliebige Material aufgebracht werden. Nach dem Aufbringen wird optional getrocknet (Verfahrensschritt c)) und anschließend kalzinieren (Verfahrenschritt b)). Auf diese Weise lassen sich mit dem bevorzugten Katalysator beschichtete Materialien/Träger bereitstellen. Solche Materialien/Träger können z. B. Metallplatten oder -gewebe, wie sie als Einbauten in Reaktoren oder Kolonnen, insbesondere Reaktivdestillationskolonnen verwendet werden können, oder auch Metall-, Glas- oder Keramikkugeln oder Kugeln von anorganischen Oxiden sein.

In einer achten Ausführungsform des Herstellverfahrens können Extrudate des Katalysators, insbesondere des erfindungsgemäßen Katalysators, dadurch erhalten werden, dass in Verfahrensschritt a) ein Alumosilikatpulver, saure wässrige Alkali- und/oder Erdalkalimetallsalzlösung, Bindemittel beispielsweise Disperal und weitere für die Extrusion übliche Formhilfsmittel beispielsweise Tone wie Bentonit oder Attapulgit in einem Kneter oder Eirich-Mischer gemischt werden und in einem Extruder zu Extrudaten vorzugsweise mit einem mittleren Durchmesser von 0,5 bis 10 mm, bevorzugt von 1 bis 5 mm und besonders bevorzugt von 1 bis 3 mm extrudiert werden und die erhaltenen feuchten Extrudate optional in Verfahrensschritt c) getrocknet und anschließend in Verfahrensschritt b) im Gasstrom kalziniert werden.

Das erfindungsgemäße Verfahren zur Herstellung von Isoolefinen, insbesondere von Isobuten, durch Spaltung von tertiären Alkoholen oder Alkyl-tert.-alkylethern in der Gasphase an einem festen Katalysator kann in allen geeigneten Reaktoren durchgeführt werden, die eine den Katalysator aufweisende Reaktionszone (Katalysatorzone) aufweisen, die räumlich von einem Heizmantel, durch den der Wärmeträger strömt, getrennt ist. Vorzugsweise wird das erfindungsgemäße Verfahren in einem Plattenreaktor, in einem Rohrreaktor, in mehreren parallel zueinander geschalteten Rohrreaktoren oder Plattenreaktoren oder in einem Rohrbündelreaktor durchgeführt. Bevorzugt wird das erfindungsgemäße Verfahren in einem Rohrbündelreaktor durchgeführt.

Es sei darauf hingewiesen, dass die Hohlkörper, in denen sich der Katalysator befindet, nicht nur Rohre im üblichen Sprachgebrauch sein müssen. Die Hohlkörper können auch nicht kreisförmige Querschnitte aufweisen. Sie können beispielsweise elliptisch oder dreieckig sein.

Die für den Bau des Reaktors verwendeten Materialien, insbesondere das Material, welches die Reaktionszone von dem Heizmantel trennt, weist vorzugsweise einen hohen Wärmeleitfähigkeitskoeffizienten (größer 40 W/(m • K)) auf. Bevorzugt wird als Material mit hohem Wärmeleitfähigkeitskoeffizient Eisen oder eine Eisenlegierung, wie z. B. Stahl eingesetzt.

Wird das erfindungsgemäße Verfahren in einem Rohrbündelreaktor durchgeführt, so weisen die einzelnen Rohre vorzugsweise eine Länge von 1 bis 15 m, bevorzugt von 3 bis 9 m und besonders bevorzugt 5 bis 9 m auf. Die einzelnen Rohre in einem im erfindungsgemäßen Verfahren eingesetzten Rohrbündelreaktor weisen vorzugsweise einen inneren Durchmesser von 10 bis 60 mm, bevorzugt von 20 bis 40 mm und besonders bevorzugt von 24 bis 35 mm auf. Es kann vorteilhaft sein, wenn die einzelnen Rohre des im erfindungsgemäßen Verfahren eingesetzten Rohrbündelreaktors eine Dicke der Rohrwand von 1 bis 4 mm, vorzugsweise von 1,5 bis 3 mm aufweisen.

In einem in dem erfindungsgemäßen Verfahren eingesetzten Rohrbündelreaktor sind die Rohre vorzugsweise parallel angeordnet. Bevorzugt sind die Rohre gleichmäßig angeordnet. Die Anordnung der Rohre kann z. B. quadratisch, dreieckig oder rautenförmig sein. Besonders bevorzugt wird eine Anordnung, bei der die virtuell verbundenen Mittelpunkte von drei gegenseitig benachbarten Rohren ein gleichseitiges Dreieck bilden, d. h. die Rohre haben den gleichen Abstand. Bevorzugt wird das erfindungsgemäße Verfahren in einem Rohrbündelreaktor, in dem die Rohre einen Abstand von 3 bis 15 mm, besonders bevorzugt von 4 bis 7 mm zueinander aufweisen, durchgeführt.

Das erfindungsgemäße Verfahren wird vorzugsweise so betrieben, dass der Umsatz an Verbindungen der Formel I im Bereich von 70 bis 98 %, insbesondere im Bereich von 90 bis 95 % liegen.

Die vorliegende Erfindung betrifft insbesondere die Herstellung von Isobuten und Methanol durch Spaltung von MTBE. Der erfindungsgemäß betriebene Reaktor kann ein integraler Bestandteil von Verfahren zur Herstellung von Isobuten aus technischem MTBE sein. Solche Verfahren sind bereits häufig im Stand der Technik beschrieben worden.

Die Spaltgemische können auf bekannte Art und Weise, z. B. so, wie im Stand der Technik beschrieben, aufgearbeitet werden. Eine bevorzugte Art der Aufarbeitung wird nachfolgend beschrieben.

Um das Spaltproduktgemisch aufzuarbeiten, kann das Spaltproduktgemisch in einem ersten Destillationsschritt in einen Isoolefin aufweisenden Kopfstrom und einen nicht umgesetzte Verbindung der Formel I aufweisenden Sumpfstrom aufgetrennt. Die destillative Auftrennung des Spaltprodukts in einen Isoolefin aufweisenden Kopfstrom und einen nicht umgesetzte Verbindung der Formel I aufweisenden Sumpfstrom erfolgt in zumindest einer Kolonne, bevorzugt in genau einer Destillationskolonne.

Eine in der destillativen Auftrennung bevorzugt eingesetzte Destillationskolonne weist vorzugsweise von 20 bis 55 theoretische Trennstufen, bevorzugt von 25 bis 45 und besonders bevorzugt von 30 bis 40 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne kann vorzugsweise zwischen 0,1 und 2,0 MPa_{(abs)} eingestellt werden. Um einen Kompressor zu sparen, kann es vorteilhaft sein, die Kolonne bei einem niedrigeren Druck als den Druck, mit dem der Spaltreaktor betrieben wird, zu betreiben. Um Isobuten gegen Kühlwasser kondensieren zu können, ist ein Druck von ca. 0,5 MPa_{(abs)} notwendig. Wird die Spaltung beispielsweise bei einem Druck von 0,65 MPa_{(abs)} betrieben, kann es vorteilhaft sein, wenn die Destillation bei einem Betriebsdruck von 0,55 bis 0,6 MPa_{(abs)} durchgeführt wird. Zur Beheizung des Verdampfers kann z. B. 0,4 MPa-Dampf eingesetzt werden. Das Sumpfprodukt enthält vorzugsweise nicht umgesetzte Verbindung der Formel I, Alkohol oder Wasser sowie gegebenenfalls Nebenprodukte, wie beispielsweise Diisobuten und/oder 2-Methoxybutan. Das Kopfprodukt ist vorzugsweise Isoolefin, insbesondere Isobuten mit einer Reinheit größer 95 Massen-%, bezogen auf das gesamte Kopfprodukt.

Optional kann die Aufarbeitung des Spaltproduktgemisches in zumindest einer als Reaktivdestillationskolonne ausgeführten Kolonne durchgeführt werden. Diese Ausführungsform des erfindungsgemäßen Verfahrens hat den Vorteil, dass der Umsatz an Verbindung der Formel I im gesamten Verfahren dadurch erhöht werden kann, dass ein Teil der in der Spaltung nicht umgesetzten Verbindung der Formel I im Reaktionsteil der Reaktivdestillationskolonne zu Isoolefin und Alkohol bzw. Wasser gespalten wird.

Als Katalysatoren können im Reaktionsteil der Reaktivdestillationskolonne alle Katalysatoren eingesetzt werden, die zur Spaltung von Verbindungen der Formel I geeignet sind. Bevorzugt werden als Katalysatoren saure Katalysatoren eingesetzt. Eine besonders bevorzugte Gruppe von sauren Katalysatoren für den Einsatz im Reaktionsteil der Reaktivdestillationskolonne sind feste, saure Ionenaustauscherharze, insbesondere solche mit Sulfonsäuregruppen. Geeignete saure Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Im Reaktionsteil der Reaktivdestillationskolonne werden die Ionenaustauscherharze in ihrer H-Form oder zumindest teilweise in der H-Form eingesetzt. Stark saure Harze des Styrol-Divinylbenzol-Typs werden u. a. unter folgenden Handelsnamen verkauft: Duolite C20, Duolite C26, Amberlyst 15, Amberlyst 35, Amberlyst 46, Amberlite IR-120, Amberlite 200, Dowex 50, Lewatit SPC 118, Lewatit SPC 108, K2611, K2621, OC 1501.

Das Porenvolumen der eingesetzten Ionenaustauscherharze beträgt vorzugsweise von 0,3 bis 0,9 ml/g, insbesondere von 0,5 bis 0,9 ml/g. Die Korngröße des Harzes beträgt vorzugsweise von 0,3 mm bis 1,5 mm, bevorzugt von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauschers beträgt, bezogen auf die Lieferform, vorzugsweise von 0,7 bis 2,0 eq/l, insbesondere von 1,1 bis 2,0 eq/l.

Im Reaktionsteil einer optional als Reaktivdestillationskolonne ausgeführten Kolonne kann der Katalysator entweder in der Packung integriert sein, wie beispielsweise in KataMax® (wie in EP 0 428 265 beschrieben) oder KataPak® (wie in EP 0 396 650 oder DE 298 07 007.3 U1 beschrieben) Packungen, oder auf Formkörpern aufpolymerisiert sein (wie in US 5,244,929 beschrieben).

Bevorzugt weist die Reaktivdestillationskolonne oberhalb der Katalysatorpackung einen Bereich rein destillativer Trennung auf. Bevorzugt weist die Zone oberhalb der Katalysatorpackung 5 bis 25, insbesondere 5 bis 15 theoretische Trennstufen auf. Die Trennzone unterhalb des Katalysators umfasst vorzugsweise von 5 bis 35, bevorzugt von 5 bis 25 theoretische Trennstufen. Der Zulauf zur Reaktivdestillationskolonne kann oberhalb oder unterhalb, vorzugsweise oberhalb der Katalysatorzone erfolgen.

Die Umsetzung der Verbindung der Formel I zu Isoolefin und Alkohol bzw. Wasser erfolgt in der Reaktivdestillation vorzugsweise in einem Temperaturbereich von 60 bis 140 °C, bevorzugt bei 80 bis 130 °C, besonders bevorzugt bei 90 bis 110 °C (Temperatur im Bereich der Kolonne, in der sich der Katalysator befindet; die Sumpftemperatur kann deutlich höher liegen).

Für den Betriebsdruck der Reaktivdestillationskolonne können prinzipiell ähnliche Betriebsbedingungen wie für die oben beschriebene Ausführung als reine Destillationskolonne gewählt werden. So wird vorzugsweise ein Betriebsdruck der Reaktivdestillationskolonne von 0,1 bis 1,2 MPa_{(abs)} eingestellt. Um einen Kompressor zu sparen, kann es vorteilhaft sein, die Kolonne bei einem niedrigeren Druck als den Druck, mit dem der Spaltreaktor betrieben wird, zu betreiben. Um Isobuten gegen Kühlwasser kondensieren zu können, ist ein Druck von ca. 0,5 MPa_{(abs)} notwendig. Wird die Spaltung beispielsweise bei einem Druck von 0,65 MPa_{(abs)} betrieben, kann es vorteilhaft sein, wenn die Reaktivdestillation mit einem Betriebsdruck von 0,55 bis 0,6 MPa_{(abs)} durchgeführt wird. Zur Beheizung des Verdampfers kann z. B. 0,4 MPa-Dampf eingesetzt werden.

Die hydraulische Belastung in der katalytischen Packung der Kolonne beträgt vorzugsweise von 10 % bis 110 %, bevorzugt von 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden (Vauck/Müller, "Grundoperationen chemischer Verfahrenstechnik", S. 626, VEB Deutscher Verlag für Grundstoffindustrie).

Auch bei einer Ausführung der Kolonne als Reaktivdestillationskolonne wird vorzugsweise ein Sumpfprodukt erhalten, das nicht umgesetzte Verbindung der Formel I und Alkohol bzw. Wasser sowie gegebenenfalls Nebenprodukte, wie beispielsweise Diisoolefin enthält. Das Kopfprodukt weist vorzugsweise Isoolefin mit einer Reinheit größer 95 Massen-% auf.

Das Kopfprodukt, das bei dieser Destillation erhalten wird und das vorzugsweise zu größer 95 Massen-% aus Isoolefin besteht, kann direkt als Verkaufsprodukt eingesetzt werden oder weiter aufgereinigt werden.

Da Isobuten mit Methanol ein Minimum-Azeotrop bildet, enthält das im ersten Destillationsschritt erhaltene Kopfprodukt neben dem Hauptprodukt Isobuten insbesondere Methanol. Als weitere Komponenten können im Kopfprodukt z. B. Dimethylether, welches z. B. durch Kondensation von Methanol entstanden sein kann, und lineare Butene (1-Buten, cis-2-Buten, trans-2-Buten), welches z. B. durch Zersetzung von 2-Methoxybutan entstanden sein können, und Wasser enthalten sein.

Aus dem Kopfprodukt kann ein Teil des Dimethylethers optional bereits im Destillationsschritt abgetrennt werden, indem der Kondensator an der Destillationskolonne bzw. Reaktivdestillationskolonne als Partialkondensator betrieben wird. In diesem kann die im Kopfprodukt enthaltene Fraktion kondensiert werden und ein Teil des im Kopfprodukt enthaltenen Dimethylethers gasförmig abgezogen werden.

Marktgängige Isoolefinqualitäten insbesondere Isobutenqualitäten sind üblicherweise praktisch frei von Alkohol, insbesondere Methanol. Methanol kann aus dem im ersten Destillationsschritt erhaltenen Kopfprodukt nach an sich bekannten Verfahren, beispielsweise durch Extraktion, abgetrennt werden. Die Extraktion von Methanol aus dem Kopfprodukt kann beispielsweise mit Wasser oder einer wässrigen Lösung als Extraktionsmittel z. B. in einer Extraktionskolonne durchgeführt werden. Vorzugsweise wird die Extraktion mit Wasser oder einer wässrigen Lösung in einer Extraktionskolonne durchgeführt, die vorzugsweise 4 bis 16 theoretische Trennstufen aufweist. Vorzugsweise wird das Extraktionsmittel in Bezug auf den zu extrahierenden Strom im Gegenstrom durch die Extraktionskolonne geführt. Die Extraktion wird vorzugsweise bei einer Temperatur von 15 bis 50 °C, bevorzugt 25 bis 40 °C durchgeführt. Beispielweise kann bei der Verwendung einer Extraktionskolonne mit mehr als 6 theoretischen Trennstufen, die bei einem Druck von 0,9 MPa_{(abs)} und einer Temperatur von 40 °C betrieben wird, ein wassergesättigtes Isoolefin, insbesondere Isobuten mit einem Isoolefingehalt bzw. Isobutengehalt von über 99 Massen-% erhalten werden.

Der bei der Extraktion erhaltene methanolhaltige Wasserextrakt kann destillativ in Wasser und Methanol getrennt werden. Das Wasser kann als Extraktionsmittel in die Extraktionsstufe zurückgeführt werden. Das Methanol kann für übliche technische Synthesen, beispielsweise Veresterungen oder Veretherungen genutzt werden.

Der feuchte Isoolefinstrom bzw. Isobutenstrom aus der Extraktionskolonne kann in einer weiteren Destillationskolonne durch Abtrennung von Wasser und optional von Dimethylether zu trockenem Isoolefin bzw. Isobuten aufgearbeitet werden. Das trockene Isoolefin bzw. Isobuten wird dabei als Sumpfprodukt erhalten. Im Kondensationssystem am Kopf der Kolonne kann nach einer Phasentrennung Wasser flüssig und Dimethylether gasförmig abgezogen werden. Eine für die Trocknung bevorzugt eingesetzte Destillationskolonne weist vorzugsweise von 30 bis 80 theoretische Trennstufen, bevorzugt von 40 bis 65 theoretische Trennstufen auf. Das Rücklaufverhältnis beträgt, in Abhängigkeit von der realisierten Stufenzahl und der erforderlichen Reinheit des Isoolefins bzw. Isobutens, vorzugsweise kleiner 60, bevorzugt kleiner 40. Der Betriebsdruck dieser für die Trocknung eingesetzten Destillationskolonne kann vorzugsweise zwischen 0,1 und 2,0 MPa_{(abs)} eingestellt werden.

Eine Aufbereitung von Isobuten durch Extraktion und Destillation ist z. B. in DE 102 38 370 ausführlich beschrieben. Bevorzugt wird aus dem im ersten Destillationsschritt erhaltenen, Isoolefin bzw. Isobuten aufweisenden Kopfstrom Methanol durch Extraktion und aus dem extrahierten Isoolefin bzw. Isobuten Dimethylether und Wasser destillativ abgetrennt.

Wird mit dem erfindungsgemäßen Verfahren Isobuten hergestellt, so kann dieses z. B. zur Herstellung von Methallylchlorid, Methallylsulfonaten, Methacrylsäure oder Methylmethacrylat eingesetzt werden. Insbesondere kann es vorteilhaft sein, wenn sowohl das Methanol als auch das Isobuten aus dem Spaltprodukt abgetrennt wird, sowohl das Methanol als auch das Isobuten zur Herstellung von Methylmethacrylat einzusetzen. Ein solches Verfahren zur Herstellung von Methylmethacrylat wird beispielsweise in EP 1 254 887 beschrieben, auf welches ausdrücklich verwiesen wird.

Das im ersten Destillationsschritt erhaltene Sumpfprodukt enthält die in der Spaltung nicht umgesetzte Verbindung der Formel I (z. B. MTBE) und den größten Teil des bei der Spaltung der Verbindung der Formel I entstandenen Alkohols bzw. Wassers. Das Sumpfprodukt kann direkt in die Spaltung zurückgeführt oder aber in einem zweiten Destillationsschritt aufgearbeitet werden. Vorzugsweise wird aus dem Sumpfprodukt des ersten Destillationsschritts in einem zweiten Destillationsschritt der größte Teil des Alkohols destillativ abgetrennt und der Rest zumindest teilweise in die Spaltung zurückgeführt.

Werden in dem erfindungsgemäßen Verfahren Kolonnen eingesetzt so können diese mit Einbauten, die z. B. aus Böden, rotierenden Einbauten, regellosen Schüttungen und/oder geordneten Packungen sind, versehen sein.

Bei den Kolonnenböden können z. B. folgende Typen zum Einsatz kommen:
Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten kann der Rücklauf z. B. durch rotierende Trichter versprüht oder mit Hilfe eines Rotors als Film auf einer beheizten Rohrwand ausgebreitet werden.

In dem erfindungsgemäßen Verfahren können, wie bereits gesagt, Kolonnen eingesetzt werden, die regellose Schüttungen mit verschiedenen Füllkörpern aufweisen. Die Füllkörper können aus fast allen Werkstoffen, insbesondere aus Stahl, Edelstahl, Kupfer, Kohlenstoff, Steingut, Porzellan, Glas oder Kunststoffen, bestehen und die verschiedensten Formen, insbesondere die Form von Kugeln, Ringen mit glatten oder profilierten Oberflächen, Ringen mit Innenstegen oder Wanddurchbrüchen, Drahtnetzringen, Sattelkörper und Spiralen aufweisen.

Packungen mit regelmäßiger/geordneter Geometrie können z. B. aus Blechen oder Geweben bestehen. Beispiele für solche Packungen sind Sulzer Gewebepackungen BX aus Metall oder Kunststoff, Sulzer Lamellenpackungen Mellapak aus Metallblech, Hochleistungspackungen von Sulzer wie Mella-pakPlus, Strukturpackungen von Sulzer (Optiflow), Montz (BSH) und Kühni (Rombopak).

Das nach dem erfindungsgemäßen Verfahren erhaltene Isobuten kann für die in der Einleitung genannten Zwecke genutzt werden.

Die nachfolgenden Beispiele sollen die Erfindung erläutern, ohne die Anwendungsbreite einzuschränken, die sich aus der Beschreibung und den Patentansprüchen ergibt.

### Beispiele:

### Beispiel a: Herstellung eines Alumosilikatformkörpers

500 g Alumosilikatpulver (Hersteller: Grace Davison, Typ: Davicat O 701, formaler Al₂O₃-Gehalt: 13 Massen-%, formaler SiO₂-Gehalt: 76 Massen-%, formaler Na₂O-Gehalt: 0,1 Massen-%, Glühverlust bei 850 °C: ca. 11 %), 363 g Disperal-Gel (formaler Al₂O₃-Gehalt: 15,6 %), welches durch Einrühren von 197 g Disperal, einem Boehmit mit einem formalen Al₂O₃-Gehalt von 77 Massen-%, der Sasol Deutschland GmbH in 803 g einer 1,28 Massen-%igen, wässrigen Salpetersäure, anschließendes gründliches Rühren, bei dem das sich bildende Gel ständig geschert und so in einem fließfähigen Zustand gehalten wird, in einem abgedeckten Behälter für 3 h bei 60 °C, Abkühlen des Gels auf Raumtemperatur und Ersetzen von eventuell verdunstetem Wasser erhalten wird, und 370 g vollentsalztes Wasser (VE-Wasser) wurden zunächst gründlich miteinander in einem Intensiv-Mischer der Firma Eirich vermischt. Anschließend erfolgte eine Pelletierung in dem Intensiv-Mischer der Firma Eirich, bei der innerhalb von 30-40 Minuten gleichmäßig rundliche Pellets mit einem Durchmesser von ca. 1 bis 3 mm erhalten wurden. Die feuchten Pellets wurden zunächst bei 120 °C im Luftstrom getrocknet und anschließend mit 2 K/min auf 550 °C aufgeheizt und bei dieser Temperatur für 10 h im Luftstrom kalziniert. Die so hergestellten Alumosilikatpellets enthielten formal 76 Massen-% Al₂O₃ und 24 Massen-% SiO₂. Weiterhin enthielt der hergestellte Katalysator 0,12 Massen-% Natriumverbindungen (berechnet als Natriumoxid). Die Zusammensetzung der Alumosilikatpellets wurde berechnet aus der Menge und der Zusammensetzung der Ausgangssubstanzen. Die Alumosilikatpellets wiesen ein Porenvolumen, bestimmt mit der oben beschriebenen Cyclohexanmethode, von 1,15 ml/g auf.

### Beispiel b: Herstellung eines geformten Katalysators (gemäß der Erfindung)

Aus VE-Wasser und Magnesiumnitrat-Hexahydrat wurde eine Imprägnierlösung mit einem Magnesiumgehalt von 4,7 Massen-% hergestellt. Der pH-Wert dieser Lösung betrug 5,1. Über eine Vakuumimprägnierung wurden eine ausgesiebte Fraktion des in Beispiel 1 hergestellten Alumosilikatträgers (Durchmesser: 1,0 mm - 2,8 mm) mit der sauren Magnesiumnitratlösung getränkt. Dazu wurden die Pellets in ein Glasrohr gefüllt und dieses für etwa 30 min evakuiert (Wasserstrahlpumpenvakuum von ca. 25 hPa). Anschließend wurde die Imprägnierlösung von unten bis über den oberen Rand der Festkörperschüttung gesaugt. Nach einer Einwirkzeit von etwa 15 Minuten wurde die Lösung, die nicht von dem Träger aufgenommen worden war, abgelassen. Die feuchten Pellets wurden zunächst im Luftstrom bei 140 °C bis zur Gewichtskonstanz getrocknet und anschließend mit 3 K/min auf 450 °C aufgeheizt und bei dieser Temperatur für 12 h kalziniert. Der hergestellte Katalysator bestand formal aus 68 Massen-% Siliziumdioxid, aus 21 Massen-% Aluminiumoxid und aus 11 Massen-% Magnesiumoxid. Weiterhin enthielt der hergestellte Katalysator 0,11 Massen-% Natriumverbindungen (berechnet als Natriumoxid). Die Zusammensetzung des Katalysators wurde berechnet aus der Menge und der Zusammensetzung der Ausgangssubstanzen sowie der abgelaufenen Imprägnierlösung. Die Natriummengen waren Bestandteil des in Beispiel 1 eingesetzten Alumosilikats. Das Porenvolumen, bestimmt mit der oben beschrieben Cyclohexanmethode, betrug 1,1 ml/g.

### Beispiel 1 (Vergleichsbeispiel)

In einem mit einem Doppelmantel ausgestatteten Laborrohrreaktor aus Edelstahl wurden Versuche zum Langzeittest des Katalysators durchgeführt. Der Reaktor wurde mit einem Katalysator gemäß Beispiel b mit einer Korngrößenverteilung zwischen 1 und 2,8 mm komplett befüllt. Dies entsprach einer Katalysatormasse von 283 g. Für die Beheizung des Reaktionsmediums wurde Marlotherm SH der Fa. Sasol Olefins & Surfactants GmbH eingesetzt. Das Edukt wurde dabei durch das Rohr und das Marlotherm SH durch den Mantel in Gleichstrom geführt. Die Versuche wurden unter Einstellung der in Tabelle 1 aufgeführten Bedingungen durchgeführt.

**Tabelle 1: Versuchbedingungen in Beispiel 1**

| | | |
|---|---|---|
| Marlotherm-Eintrittstemperatur | [°C] | 220 |
| Marlotherm-Austrittstemperatur | [°C] | 218-219 |
| Edukteintrittstemperatur | [°C] | 220 |
| WHSV | [h⁻¹] | 9,6 |
| Druck | [MPa] | 0,7 |

| | | |
|---|---|---|
| WHSV = weight hourly space velocity | | |

Unter diesen Bedingungen wurde für den MTBE-Umsatz ein Anfangswert von 22 % und eine minimale Temperatur im Rohrinneren von 164 °C erreicht. Die Selektivität zu Isobuten lag bei 99,99 %. Während 4000 Stunden verringerte sich der Umsatz auf 2,5 % bei gleich bleibender Selektivität.

### Beispiel 2 (erfindungsgemäß)

In einem mit einem Doppelmantel ausgestatteten Laborrohrreaktor aus Edelstahl wurden Versuche zum Langzeittest des Katalysators durchgeführt. Der Reaktor wurde mit einem Katalysator gemäß Beispiel b mit einer Korngrößenverteilung zwischen 1 und 2,8 mm komplett befüllt. Dies entsprach einer Katalysatormasse von 283 g. Für die Beheizung des Reaktionsmediums wurde Marlotherm SH der Sasol Olefins & Surfactants GmbH im Gleichstrom im Doppelmantel eingesetzt. Die Versuche wurden unter Einstellung der in Tabelle 2 aufgeführten Bedingungen durchgeführt.

**Tabelle 2: Versuchbedingungen in Beispiel 2**

| | | |
|---|---|---|
| Marlotherm-Eintrittstemperatur | [°C] | 250 |
| Marlotherm-Austrittstemperatur | [°C] | 250 |
| Edukteintrittstemperatur | [°C] | 250 |
| WHSV | [h⁻¹] | 1,6 |
| Druck | [MPa] | 0,7 |

Unter diesen Bedingungen wurde für den MTBE-Umsatz ein Anfangswert von 85 % und eine minimale Temperatur im Rohrinneren von 218 °C erreicht. Die Selektivität zu Isobuten lag bei 99,98 %. Während 4000 Stunden verringerte sich der Umsatz auf 70 % bei gleich bleibender Selektivität.

Der Vergleich der Ergebnisse von Beispiel 1 und 2 zeigten, dass durch die erfindungsgemäße Verfahrensweise die Standdauer des Katalysators deutlich erhöht werden konnte.

## Patentansprüche

1. Kontinuierliches Verfahren zur Herstellung von Isoolefinen mit 4 bis 6 C-Atomen durch Spaltung von Verbindungen der Formel **I**
R₁-O-R₂ (**I**)
mit R₁ = einem 4 bis 6 Kohlenstoffatome aufweisenden tertiären Alkylrest und R₂ = H oder einem Alkylrest, in der Gasphase an einem festen Katalysator im Temperaturbereich 200 bis 400 °C bei einem Druck von 0,1 bis 1,2 MPa in einem Reaktor, der mit einem Heizmantel ausgestattet ist und der mit einem flüssigen Wärmeträger beheizt wird, **dadurch gekennzeichnet,**
**dass** die Spaltung so durchgeführt wird, dass der Temperaturabfall in der Katalysatorzone an jeder beliebigen Stelle in Bezug auf die Eingangstemperatur kleiner 50 °C beträgt, dass das Reaktionsgemisch im Reaktor und der Wärmeträger im Mantel im Gleichstrom durch den Reaktor strömen und dass die Temperaturdifferenz des Wärmeträgers zwischen Zulaufstelle zum Reaktor und Ablauf aus dem Reaktor auf weniger als 40 °C eingestellt wird.

2. Verfahren nach Anspruch 1,
**dadurch gekennzeichnet,**
**dass** als Verbindungen der Formel I tert.-Butanol, Methyl-tert.-butylether, Ethyl-tert.-butylether und/oder tert.-Amylmethylether eingesetzt werden.

3. Verfahren nach Anspruch 1 oder 2,
**dadurch gekennzeichnet,**
**dass** ein Gemisch von zumindest zwei Verbindungen der Formel I eingesetzt wird.

4. Verfahren nach Anspruch 3,
**dadurch gekennzeichnet,**
**dass** als Gemisch von Verbindungen der Formel I ein Gemisch, welches tert.-Butanol und Methyl-tert.-butylether aufweist, eingesetzt wird.

5. Verfahren nach zumindest einem der Ansprüche 1 bis 4,
**dadurch gekennzeichnet,**
**dass** der Temperaturabfall in der Katalysatorzone kleiner 30 °C beträgt.

6. Verfahren nach zumindest einem der Ansprüche 1 bis 5,
**dadurch gekennzeichnet,**
**dass** der Wärmeträger mit einer Temperatur, die 10 bis 30 °C höher ist als die Temperatur des in den Reaktor strömenden Edukts, in den Heizmantel des Reaktors geleitet wird.

7. Verfahren nach zumindest einem der Ansprüche 1 bis 6,
**dadurch gekennzeichnet,**
**dass** als feste Katalysatoren Metalloxide, Metallmischoxide, Säuren auf Metalloxidträgern oder Metallsalze oder Mischungen davon eingesetzt werden.

8. Verfahren nach Anspruch 7,
**dadurch gekennzeichnet,**
**dass** feste Katalysatoren eingesetzt werden, die eine mittlere Korngröße von 2 bis 4 mm aufweisen.

9. Verfahren nach zumindest einem der Ansprüche 1 bis 8,
**dadurch gekennzeichnet,**
**dass** wenn als Verbindung I MTBE eingesetzt wird, dieses an einem Katalysator, der aus Magnesiumoxid, Aluminiumoxid und Siliziumoxid besteht, zu Isobuten und Methanol gespaltet wird.

10. Verfahren nach zumindest einem der Ansprüche 1 bis 9,
**dadurch gekennzeichnet,**
**dass** das Verfahren in einem Rohrreaktor, Rohrbündelreaktor oder Plattenreaktor durchgeführt wird.

11. Verfahren nach Anspruch 10,
**dadurch gekennzeichnet,**
**dass** das Verfahren in einem Rohrbündelreaktor durchgeführt wird.

12. Verfahren nach Anspruch 10 oder 11,
**dadurch gekennzeichnet,**
**dass** das Verfahren in einem Rohrbündelreaktor, dessen einzelne Rohre eine Länge von 1 bis 15 m aufweisen, durchgeführt wird.

13. Verfahren nach zumindest einem der Ansprüche 10 bis 12,
**dadurch gekennzeichnet,**
**dass** das Verfahren in einem Rohrbündelreaktor, dessen einzelne Rohre einen inneren Durchmesser von 10 bis 60 mm aufweisen, durchgeführt wird.

14. Verfahren nach zumindest einem der Ansprüche 10 bis 13,
**dadurch gekennzeichnet,**
**dass** das Verfahren in einem Rohrbündelreaktor, dessen einzelne Rohre eine Dicke der Rohrwand von 1 bis 4 mm aufweisen, durchgeführt wird.

15. Verfahren nach zumindest einem der Ansprüche 10 bis 14,
**dadurch gekennzeichnet,**
**dass** das Verfahren in einem Rohrbündelreaktor, in dem die Rohre einen Abstand von 3 bis 15 mm zueinander aufweisen, durchgeführt wird.

## Claims

1. A continuous process for preparing isoolefins having 4 to 6 carbon atoms by cleaving compounds of the formula I
R₁-O-R₂ (I)
where R₁ = a tertiary alkyl radical having 4 to 6 carbon atoms and R₂ = H or an alkyl radical in the gas phase over a solid catalyst in the temperature range of 200 to 400°C at a pressure of 0.1 to 1.2 MPa in a reactor which is equipped with a heating jacket and is heated with a liquid heat carrier,
**characterized in that**
the cleavage is carried out in such a way that the temperature drop in the catalyst zone at any point in relation to the entrance temperature is less than 50°C, **in that** the reaction mixture in the reactor and the heat carrier in the jacket flow through the reactor in cocurrent, and **in that** the temperature difference of the heat carrier between the feed point to the reactor and outlet from the reactor is adjusted to less than 40°C.

2. A process according to claim 1,
**characterized in that**
the compounds of the formula I used are tert-butanol, methyl tert-butyl ether, ethyl tert-butyl ether and/or tert-amyl methyl ether.

3. A process according to either of claims 1 and 2,
**characterized in that**
a mixture of at least two compounds of the formula I is used.

4. A process according to claim 3,
**characterized in that**
the mixture of compounds of the formula I used is a mixture which comprises tert-butanol and methyl tert-butyl ether.

5. A process according to at least one of claims 1 to 4,
**characterized in that**
the temperature drop in the catalyst zone is less than 30°C.

6. A process according to at least one of claims 1 to 5,
**characterized in that**
the heat carrier is passed into the heating jacket of the reactor at a temperature which is 10 to 30°C higher than the temperature of the reactant flowing into the reactor.

7. A process according to at least one of claims 1 to 6,
**characterized in that**
the solid catalysts used are metal oxides, mixed metal oxides, acids on metal oxide supports or metal salts or mixtures thereof.

8. A process according to claim 7,
**characterized in that**
solid catalysts are used which have a mean particle size of 2 to 4 mm.

9. A process according to at least one of claims 1 to 8,
**characterized in that**
when the compound I used is MTBE, it is cleaved over a catalyst which comprises magnesium oxide, aluminium oxide and silicon oxide to give isobutene and methanol.

10. A process according to at least one of claims 1 to 9,
**characterized in that**
the process is performed in a tubular reactor, tube bundle reactor or plate reactor.

11. A process according to claim 10,
**characterized in that**
the process is performed in a tube bundle reactor.

12. A process according to either of claims 10 and 11,
**characterized in that**
the process is performed in a tube bundle reactor whose individual tubes have a length of 1 to 15 m.

13. A process according to at least one of claims 10 to 12,
**characterized in that**
the process is performed in a tube bundle reactor whose individual tubes have an internal diameter of 10 to 60 mm.

14. A process according to at least one of claims 10 to 13,
**characterized in that**
the process is performed in a tube bundle reactor whose individual tubes have a thickness of the tube wall of 1 to 4 mm.

15. A process according to at least one of claims 10 to 14,
**characterized in that**
the process is performed in a tube bundle reactor in which the tubes have a separation of 3 to 15 mm from one another.

## Revendications

1. Procédé continu pour la préparation d'isooléfines comprenant 4 à 6 atomes de carbone par dissociation de composés de formule I
R₁-O-R₂ (I)
dans laquelle
R₁ = représente un radical alkyle tertiaire présentant 4 à 6 atomes de carbone et
R₂ = représente H ou un radical alkyle, en phase gazeuse sur un catalyseur solide dans une plage de température de 200 à 400°C à une pression de 0,1 à 1,2 MPa dans un réacteur qui est équipé d'une enveloppe chauffante et qui est chauffée par un agent caloporteur liquide,
**caractérisé en ce que** la dissociation est réalisée de manière telle que la chute de température dans la zone catalytique en n'importe quel endroit, par rapport à la température à l'entrée, est inférieure à 50°C, **en ce que** le mélange réactionnel dans le réacteur et l'agent caloporteur dans l'enveloppe s'écoulent en courant parallèle au travers du réacteur et **en ce que** la différence de température de l'agent caloporteur entre le site d'introduction dans le réacteur et l'évacuation du réacteur est réglée à moins de 40°C.

2. Procédé selon la revendication 1, **caractérisé en ce qu'**on utilise, comme composés de formule I, du tert-butanol, du méthyl-tert-butyléther, de l'éthyl-tert- butyléther et/ou du tert-amylméthyléther.

3. Procédé selon la revendication 1 ou 2, **caractérisé en ce qu'**on utilise un mélange d'au moins deux composés de formule I.

4. Procédé selon la revendication 3, **caractérisé en ce qu'**on utilise, comme mélange de composés de formule I, un mélange qui présente du tert-butanol et du méthyl-tert-butyléther.

5. Procédé selon au moins l'une quelconque des revendications 1 à 4, **caractérisé en ce que** la chute de température dans la zone catalytique est inférieure à 30°C.

6. Procédé selon au moins l'une quelconque des revendications 1 à 5, **caractérisé en ce que** l'agent caloporteur est guidé dans l'enveloppe chauffante du réacteur à une température qui est supérieure de 10 à 30°C à la température du produit de départ s'écoulant dans le réacteur.

7. Procédé selon au moins l'une quelconque des revendications 1 à 6, **caractérisé en ce qu'**on utilise, comme catalyseurs solides, des oxydes métalliques, des oxydes mixtes de métaux, des acides sur des supports d'oxyde métallique ou des sels métalliques ou des mélanges de ceux-ci.

8. Procédé selon la revendication 7, **caractérisé en ce qu'**on utilise des catalyseurs solides, qui présentent une grosseur moyenne de particule de 2 à 4 mm.

9. Procédé selon au moins l'une quelconque des revendications 1 à 8, **caractérisé en ce que**, lorsqu'on utilise comme composé I du MTBE, celui-ci est dissocié sur un catalyseur, qui est constitué d'oxyde de magnésium, d'oxyde d'aluminium et d'oxyde de silicium, en isobutène et en méthanol.

10. Procédé selon au moins l'une quelconque des revendications 1 à 9, **caractérisé en ce que** le procédé est réalisé dans un réacteur tubulaire, un réacteur à faisceau tubulaire ou un réacteur à plaques.

11. Procédé selon la revendication 10, **caractérisé en ce que** le procédé est réalisé dans un réacteur à faisceau tubulaire.

12. Procédé selon la revendication 10 ou 11, **caractérisé en ce que** le procédé est réalisé dans un réacteur à faisceau tubulaire, dont les différents tubes présentent une longueur de 1 à 15 m.

13. Procédé selon au moins l'une quelconque des revendications 10 à 12, **caractérisé en ce que** le procédé est réalisé dans un réacteur à faisceau tubulaire, dont les différents tubes présentent un diamètre interne de 10 à 60 mm.

14. Procédé selon au moins l'une quelconque des revendications 10 à 13, **caractérisé en ce que** le procédé est réalisé dans un réacteur à faisceau tubulaire, dont les différents tubes présentent une épaisseur de la paroi tubulaire de 1 à 4 mm.

15. Procédé selon au moins l'une quelconque des revendications 10 à 14, **caractérisé en ce que** le procédé est réalisé dans un réacteur à faisceau tubulaire, dans lequel les tubes présentent une distance de 3 à 15 mm les uns des autres.
